# EUROPEAN PATENT APPLICATION

(11) **EP 4 613 260 A1**
(43) Date of publication of application: **10.09.2025**
(21) Application number: 23883925.2
(22) Date of filing: 31.10.2023
(51) Int. Cl.: A61K 8/64, A61Q 19/08

(54) **COMPOUNDS AND COSMETIC, DERMOCOSMETIC, COSMECEUTICAL OR PHARMACEUTICAL COMPOSITIONS FOR RELAXING MUSCLES, INCLUDING THE SKELETAL MUSCLES OF THE FACE, FOR ANTI-AGING COSMETIC ACTION, FOR REDUCING AND/OR SMOOTHING EXPRESSION LINES AND WRINKLES AND/OR FOR ANTIPERSPIRANT ACTION, USES AND METHODS**

(30) Priority: 31.10.2022 BR 102022022131
(71) Applicant: Chemyunion Ltda, 18087-800 Sorocaba (BR)
(72) Inventor: KATEKAWA, Edson, Sorocaba (BR); ROGERIO PRINCIVAL, Cleverson, Sorocaba (BR); MUSSI, Lilian, Sorocaba (BR); VIDAL MAGALHÃES, Wagner, Sorocaba (BR)
(74) Representative: Hoffmann Eitle
(86) International application number: PCT/BR2023/050369
(87) International publication number: WO 2024/092334

(57) **Abstract**

The present invention describes new compounds and compositions effective for relaxing muscles, including skeletal muscles of the face, for anti-aging cosmetic action, for reducing and/or smoothing expression lines and wrinkles and/or for antiperspirant action.

## Description

### Technical Field of the Invention

The present patent application describes new compounds and compositions effective for muscle relaxation, including skeletal muscles of the face, for anti-aging cosmetic action, for reducing and/or smoothing expression lines and wrinkles and/or for antiperspirant action. The present invention is in the technical fields of cosmetic, dermocosmetic, cosmeceutical and pharmaceutical treatments.

### Background of the Invention

Wrinkles and expression lines on the skin are interpreted as visible signs of age advancement in individuals. Although morphological patterns vary according to the region they affect, wrinkles tend to develop perpendicular to the direction of underlying muscle contraction (Lemperle, G., 2001. A classification of facial wrinkles. Plast. Reconstr. Surg. 106, 1733-1734).

Wrinkles can be classified into two main types: dynamic and static. Dynamic wrinkles appear at all ages at the time of facial expression, that is, the underlying skeletal muscles contract, causing great compressive tensions and mechanical curvature of the skin (Mazza, E., Papes, O., Rubin, M., Bodner, S., Binur, N., 2007. Simulation of the aging face. J. Biomech. Eng. 129, 619-623). The level of contraction employed determines not only the shape but also the depth of such expression lines (Hara, Y., Hirao, T., Iwai, I., 2017. Facial expression under stiff stratum corneum leads to strain concentrations, followed by residual wrinkle formation. Int. J. Cosmet. Sci . 39, 66-71). Similarly, when the underlying musculature relaxes, these marks disappear. Static (or permanent) wrinkles, in turn, persist regardless of muscle relaxation and their severity and incidence are directly associated with advancing age (Imokawa, G., 2008. Recent advances in characterizing biological mechanisms underlying UV-induced wrinkles: a pivotal role of fibrobrast-derived elastase. Arch. Dermatol. Res. 300, 7-20; Hara, Y., Hirao, T., Iwai, I., 2017. Facial expression under stiff stratum corneum leads to strain concentrations, followed by residual wrinkle formation. Int. J. Cosmet. Sci. 39, 66-71). The transition from dynamic to static wrinkles may arise from damage resulting from frequent muscle contractions.

A useful strategy for reducing the intensity of expression lines and wrinkles consists of directly discouraging muscle action or containing the activity of the innervating neuron.

In humans, as in all mammals, skeletal muscle fibers are innervated by nerve fibers that originate from motor neurons in the spinal cord (ADRIAN ED AND BRONK DW The discharge of impuses in motor nerve fibres. Part II: The frequency of discharge in reflex and voluntary contractions. J Physiol .119-51. 1929). In the region near their nerve ending the axons branch out on the extracellular surface of the muscle fibre forming a synapse called the neuromuscular junction (KATZ B. Nerve, muscle, and synapse. McGraw-Hill Company. 1966). Each nerve fiber can branch out and stimulate up to hundreds of muscle fibers (ADRIAN ED AND BRONK DW The discharge of impulses in motor nerve fibers. Part II: The frequency of discharge in reflex and voluntary contractions. J Physiol .119-51. 1929).

The neuromuscular junction consists of the connection between the nervous and muscular systems via synapse, composed of pre- and postsynaptic terminals. The presynaptic terminals store hundreds of thousands of vesicles containing the neurotransmitter acetylcholine (VAN DER KLOOT W. AND MOLGO, W. Quantal acetylcholine release at the vertebrate neuromuscular junction. Physiological Reviews. 74; 899-991. 1994). Between the presynaptic terminal and the postsynaptic terminal there is a space about 20 to 30 nanometers wide called the synaptic cleft (GOYAL RK AND ARUN C. Structure activity relationship of synaptic and junctional neurotransmission. Auton Neurosci. 176: 11-31, 2013). After being released into the synaptic cleft, the neurotransmitter can bind to nicotinic receptors located throughout the postsynaptic terminal (ZUBER B., NIKONENKO I., KLAUSER P., MULLER D. AND DUBOCHET J. The mammalian central nervous system synaptic cleft contains a high density of periodically organized complexes. PNAS. 102; 52. 2005).

In the synaptic cleft, in turn, acetylcholine binds to nicotinic cholinergic receptors located in the postsynaptic terminal of the muscle. Nicotinic acetylcholine receptors are made up of five subunits, two of the alpha type, one beta, one gamma and one more that can be delta or epsilon that form a channel (TALY A., CORRINGER PJ, GUEDIN D., LESTAGE P. AND CHANGEUX JP Nicotinic receptors: Allosteric transitions and therapeutic targets in the nervous system. Nat Rev Drug Discov. 8:733-750, 2009). Each alpha subunit has a binding site for acetylcholine. When acetylcholine units bind to both alpha subunits, a conformational change in the channel is caused, resulting in its opening, which in turn increases the conductance for cations (especially Na+ and K+). The influx of Na+ results in depolarization of the neuromuscular junction. When the depolarizations are sufficient to reach the threshold of the muscle fiber, they result in the generation of an action potential.

The muscle action potential propagates through the muscle cell membrane to the T tubules, where it activates voltage-sensitive ion channels. This activation releases Ca2+ molecules that bind to ryanodine receptors ( RyR ) on the sarcoplasmic reticulum. The opening of RyRs causes increases in the concentration of Ca2+ in the cytosol of muscle fibers. Ca2+ binds to its receptors on troponin C, producing conformational changes in the troponin -tropomyosin complex. The changes cause tropomyosin (an agent that blocks the interaction between actin and myosin) to be released, thus allowing the start of the cross-bridge cycle, causing the sliding and approximation of the actin and myosin sites, which results in muscle contraction (ALABI AA AND TSIEN RW Synaptic vesicle pools and cold dynamics. Spring Harbor Perspective Biol 2012).

One of the main ways in which such attenuation is achieved is through treatment with botulinum neurotoxin A - which considerably reduces the intensity of expression lines and wrinkles, since it strongly inhibits the release of Ca2+-dependent neurotransmitters in neurons. Botulinum neurotoxins are metalloproteases that specifically cleave synaptic proteins essential for regulated neuronal exocytosis (Johnson, EA Clostridial toxins as therapeutic agents: benefits of nature's most toxic proteins. Annu. Rev. Microbiol.53,551-575,1999), specifically the vesicular protein VAMP, and the membrane proteins syntaxin and SNAP-25. These proteins form the SNARE protein complex, which is destabilized by the action of the neurotoxin, preventing fusion of the vesicle with the plasma membrane and, consequently, abrogating Ca2+-triggered exocytosis (Chen, YA and Scheller, RH SNARE- mediated membrane fusion. Nat. Rev. Mol. Cell Biol. 2.98-106, 2001).

BTX-A (commonly known as Botox) is one of seven serotypes of botulinum toxin produced by the bacterium *Clostridium botulinum,* a Gram-positive, spore-forming anaerobe. Cosmetic uses of Botox include the treatment of vertical lines between the eyebrows (glabellar lines), periorbital lines, horizontal forehead lines, wrinkles around the mouth, and the thick platysmal bands around the neck, also known as "turkey neck" (Blitzer A, Binder WJ. 2002. Cosmetic uses of botulinum neurotoxin type A: an overview. Arch Facial Plast Surg, 4:214-20). It is most effective for wrinkles that form due to muscle contraction, and improvement in appearance occurs within 1-14 days after injection. It has its peak action at approximately 4 weeks and begins to fade after 10-12 weeks; therefore, a repeat injection is necessary approximately every 3-4 months to maintain the results obtained (Klein AW. 2005. Complications with the use of botulinum toxin. Int Ophthal Clin, 45:171-6). BTX-A is not effective for wrinkles caused by mechanisms other than muscle contraction, such as damage from environmental pollutants, photoaging, or subcutaneous soft tissue atrophy such as lipoatrophy.

Another known application for Botox is in the treatment of excessive sweating, known as hyperhidrosis. Naumann and Lowe published the results of a clinical study for bilateral axillary hyperhidrosis, with favorable results (Neumann M, Lowe NJ, Botulinum toxin type A in treatment of bilateral primary axillary hyperhidrosis: randomized, parallel group, double blind, placebo controlled trial, BMJ 2001,323,7313,596-599). Apparently, the action occurs through denervation functional of the glands sweat glands (Swartling C et al., Sweat gland morphology and periglandular innervation in essential palmar hyperhidrosis before and after treatment with intradermal botulinum toxin. J Am Acad Dermatol 2004, 51,5,739-745). The action lasts for at least 16 weeks, requiring subsequent reapplications that may become more spaced out, with an average duration that can reach 7 months (Farrell J et al., Retrospective analysis of the Efficacy and duration of botulinum toxin A injections in 30 patients with palmar hyperhidrosis. 2021, 51,9,1517-1521).

Regarding safety, although botulinum neurotoxins, especially type A, are widely used to attenuate signs of facial aging, their use is limited due to their high toxicity and, for this reason, must be under strict medical control (Benedetto, AV Environment and skin aging. Clinic. Derm. 16,129-139, 1998).

Furthermore, these toxins have side effects; the most common are related to the injection technique and include local redness, bruising, swelling, and mild pain. Patients with severe wrinkles (requiring a higher dose of the toxin) or who have altered facial anatomy are at greater risk of complications. Other side effects include eyelid ptosis, focal facial weakness, and headache. Serious adverse events may occur in the form of dysphagia, flu-like symptoms, and hypersensitivity reactions.

The search for the state of the art revealed the following patent documents that have already aimed to present solutions to such problems.

Document CN108434080 discloses a small antibacterial peptide Indolicidin: IleLeuProTrpLysTrpProTrpTrpProTrpArgArg-NH2 and used in a cosmetic composition indicated for skin regeneration and repair, including treatment of stretch marks and signs of aging. Furthermore, it indicates that an oligopeptide palmitoylated can help increase skin hydration and substantially improve the repairing effect of the composition. The aforementioned peptide is disclosed in the document as an antibacterial, the sequence does not have any of the modifications of the peptides of the present invention and is used in conjunction with other components that are indicated as having skin regeneration and repair activity.

Document BR112014023888 reveals a small peptide with the sequence ThrLysProTrpLysTrp, but for use in stimulating sexual activity. Furthermore, this document indicates that certain tetrapeptides that were tested do not have the aforementioned activity.

Document BR112020017397 discloses a small tetrapeptide LysTrpLysLys and used in a cosmetic composition indicated to reduce the appearance of wrinkles on the skin. Said peptide has a structure distinct from that disclosed by the present patent application considering both the peptide portion and possible fusion/functionalization molecules to the peptide.

Thus, this patent application aims to present alternative ways of solving the problems contained in the state of the art. From what can be inferred from the literature researched, no documents were found anticipating or suggesting the teachings of the present invention.

### Summary of the Invention

Thus, the present invention aims to solve the problems present in the state of the art through the use of new active compounds designated for muscle relaxation, including the skeletal muscles of the face, for cosmetic anti-aging purposes by smoothing expression lines and wrinkles.

The invention additionally enables the reduction/smoothing of expression lines and wrinkles through its use in topical formulations, intended for cosmetic, dermocosmetic, cosmeceutical or pharmaceutical use.

In one embodiment, the new peptides are applied for antiperspirant action, whether in cosmetic or pharmaceutical applications, by inhibiting nerve endings responsible for signaling the effector response of the sweat glands, producing the temporary cessation of excessive sweating.

Thus, a new compound is presented, comprising the general formula:

R1-AA1x-AA2y-AA3z-AA4b-R2

where:
AA1 is a side chain-containing amino acid selected from the group Ala, Ile, Leu, Val, Gly, Pro, His, Arg, Lys, norleucine, norvaline, 2-aminobutyric acid, sarcosine, citrulline, ornithine, 2,3-diaminopropionic acid, 2,4-diaminobutyric acid, and (S)-2-amino-4-guanindinobutyric acid;
AA2 is an amino acid containing an aromatic side chain, selected from the group Thr, Trp, Phe, Tyr, 1-naphthylalanine, 2-naphthylalanine and phenylglycine;
AA3 is an amino acid containing a polar side chain, selected from the group His, Arg, Lys, citrulline, ornithine, 2,3-diaminopropionic acid, 2,4-diaminobutyric acid, and (S)-2-amino-4-guanindinobutyric acid, or, Trp;
AA4 is an amino acid containing an aromatic side chain, selected from the group Trp, Phe, Tyr, 1-naphthylalanine, 2-naphthylalanine and phenylglycine;
(x, y, z, b) = 1 or 2, where the indices x, y, z, b are integers that can adopt values 1 or 2, representing the number of units of, respectively, AA1, AA2, AA3 and AA4;
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, -OR3 and - SR3, where R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
their stereoisomers, mixtures and/or their cosmetically or pharmaceutically acceptable salts.

In one embodiment of the present invention, said compound is defined by:
AA1 be Pro;
AA2 be Thr, Trp, Tyr or 1-naphthylalanine, 2-naphthylalanine and phenylglycine;
AA3 be Lys or Trp;
AA4 be Trp, Lys or 1-naphthylalanine.
where R1 is either Pal-, which identifies an N- palmitoylated peptide, or Ac-, which identifies an N-acetylated peptide.

In one embodiment of the present invention, said compound is Pro-Trp -Lys- Trp (SEQ ID NO: 1), Pal -Pro- Thr -Lys- Trp (SEQ ID NO: 2), Ac-Pro-Trp -Lys- Trp (SEQ ID NO: 3), Pro-1-naphthylAla-Lys- 1-naphthylAla (SEQ ID NO: 4), Pal - Pro- Trp -Lys- Trp (SEQ NO ID 5), Pro- Thr -Lys- Trp (SEQ NO ID 6), Ac-Pro- Thr -Lys- Trp (SEQ NO ID 7),
where the prefix Pal - identifies an N- palmitoylated peptide and the prefix Ac- identifies an N-acetylated peptide.

In one embodiment of the present invention, said compound is for relaxing muscles, including skeletal muscles of the face, for anti-aging cosmetic action, for reducing and/or smoothing expression lines and wrinkles and/or for antiperspirant action.

A new cosmetic, dermocosmetic, cosmeceutical or pharmaceutical composition comprising at least one compound as defined above is also presented.

In one embodiment of the present invention, said composition has a concentration of the compound between 0.001% (w/w) to 10.00% (w/w).

In one embodiment of the present invention, said composition is for anti-aging cosmetic action, for reducing and/or smoothing expression lines and wrinkles and/or for antiperspirant action.

A new use of said composition is also presented for the production of cosmetic, dermocosmetic, cosmeceutical or pharmaceutical formulations for the relaxation of muscles, including the skeletal muscles of the face, for anti-aging cosmetic action, for the reduction and/or smoothing of expression lines and wrinkles and/or for antiperspirant action in an individual.

A new cosmetic method for relaxing the skeletal muscles of the face, for anti-aging cosmetic action, for reducing and/or smoothing expression lines and wrinkles and/or for antiperspirant action, comprising the application of an effective cosmetic amount of a composition defined above to an individual, is also presented.

A new dermocosmetic, cosmeceutical or pharmaceutical method is also presented for relaxing the skeletal muscles of the face, for anti-aging cosmetic action, for reducing and/or smoothing expression lines and wrinkles and/or for antiperspirant action comprising the application of an effective cosmetic amount of a composition defined above to an individual.

In one embodiment of the present invention, R1 and R2 are not proteinogenic amino acids.

In the context of the present invention, stereoisomers of the active ingredients described herein, mixtures and/or their cosmetically or pharmaceutically acceptable salts are also included.

The peptides presented in this invention are safe and effective substances in reducing the intensity (area, length and depth) of wrinkles and expression lines, through the blocking action of muscular nicotinic acetylcholine receptors.

These and other objects of the invention may be immediately recognized by those skilled in the art and descriptions will be provided below in sufficient detail for their reproduction by a person skilled in the art.

### Detailed Description of the Invention

This detailed description of the invention establishes some nonlimiting definitions of the main terminologies and technical characteristics used throughout this patent application, as well as providing examples of some of the embodiments of the present invention so that it can be reproduced by a person skilled in the art.

It should be noted that for the description of amino acids in this document, 1 or 3 letter abbreviations may be used, as widely adopted by the scientific community and the International Nucleotide Sequence Database.

This invention further includes any and all conservative substitutions of the sequences described herein. Conservative substitution is understood as the replacement of one amino acid by another with similar hydrophobicity, polarity and/or side chain, so that there is little or no three-dimensional or functional alteration of the peptide.

Among these peptides, there are, as examples, Pro- Trp -Lys- Trp (SEQ NO ID 1), Pal -Pro- Thr -Lys- Trp (SEQ NO ID 2), Ac-Pro- Trp -Lys- Trp (SEQ NO ID 3), Pro- 1-naphthyl Ala-Lys- 1-naphthyl Ala (SEQ NO ID 4), Pal - Pro- Trp -Lys- Trp (SEQ NO ID 5), Pro- Thr -Lys- Trp (SEQ NO ID 6), Ac-Pro-Thr -Lys- Trp (SEQ NO ID 7) where the prefix Pal - identifies an N- palmitoylated peptide and the prefix Ac- identifies an N-acetylated peptide.

Such peptides can be obtained through various methods, preferably by chemical synthesis in solid phase or in homogeneous systems, without excluding other possibilities, such as biotechnology, hydrolysis and/or purification of proteins and other methods that may exist or may come to exist.

These peptides can be used in a concentration range between 0.001% (w/w) to 10.00% (w/w), preferably close to 0.01% (w/w), applied in a cosmetic or pharmaceutical formulation suitable for human or veterinary use.

A cosmetic or pharmaceutical formulation suitable for human or veterinary use is understood to include any and all types of solvents, dispersion media, encapsulations, permeation enhancing agents, surfactants, preservatives and other ingredients, alone or in combination, that are physiologically compatible.

Thus, the invention describes the use of peptides capable of acting on the relaxation of skeletal muscles. In one embodiment, the action occurs on the skeletal muscles of the face for anti-aging cosmetic purposes by smoothing expression lines and wrinkles.

The invention also describes the use of peptides capable of acting on the denervation of sweat glands, providing a cosmetic antiperspirant effect.

### Example 1: Assessment of clinical efficacy

Two groups of 10 volunteers underwent 28 days of topical treatment with the peptides SEQ NO ID 1 (Pro- Trp -Lys- Trp) or SEQ NO ID 2 (Pal -Pro-Thr -Lys- Trp) in the facial region. Nasolabial and glabellar lines were evaluated on Day 0 (before) and Day 28 (after 4 weeks of treatment) using Bio3D Structured -light Scanner. For wrinkle evaluation, images of the different areas were taken and processed through specific software. Three parameters (total area, length and depth of wrinkles) were obtained for each of the areas in each of the volunteers.

**Table 1. Representation of the total area, length and depth of nasolabial and glabellar wrinkles, before (Day 0) and after 28 days (Day 28) of treatment (Day 60) with formulations containing SEQ NO ID 1 (Pro- Trp -Lys - Trp) at 500 ppm and SEQ NO ID 2 (Pal -Pro- Thr -Lys- Trp) at 500 ppm.**

| | Decreased wrinkle intensity (D28/D0) | | |
|---|---|---|---|
| | Wrinkle area | Wrinkle length | Wrinkle depth |
| SEQ NO ID 1 | -10.92% | -14.97% | -9.23% |
| SEQ NO ID 2 | -7.81% | -11.76% | -6.59% |

The results expressed in Table 1 demonstrate the ability of the peptides SEQ NO ID 1 (Pro- Trp -Lys- Trp ) and SEQ NO ID 2 (Pal -Pro- Thr - Lys- Trp) to reduce the parameters depth, area and length of wrinkles.

The products can be used as cosmetics, cosmeceuticals or topical or transdermal drugs capable of promoting muscle relaxation and, consequently, reducing the intensity of wrinkles and expression lines. This list of applications includes cosmetics, creams, lotions, serums, ointments and rinse-off products, with the purpose of reducing the intensity of wrinkles on the skin and promoting anti-aging action.

The examples disclosed herein are intended to merely exemplify some of the numerous forms of realization and use of the present invention, without limiting the interpretation of its scope and breadth, as well as possible alternative forms and configurational variations thereof that will be defined based on the claims presented herein.

## Claims

1. Compound, **characterized by** comprising the general formula:
R1 -AA1x-AA2y-AA3z-AA4b-R2
wherein:
AA1 is a side chain-containing amino acid selected from the group Ala, Ile, Leu, Val, Gly, Pro, His, Arg, Lys, norleucine, norvaline, 2-aminobutyric acid, sarcosine, citrulline, ornithine, 2,3-diaminopropionic acid, 2,4-diaminobutyric acid, and (S)-2-amino-4-guanindinobutyric acid;
AA2 is an amino acid containing an aromatic side chain, selected from the group Thr, Trp, Phe, Tyr, 1-naphthylalanine, 2-naphthylalanine and phenylglycine;
AA3 is an amino acid containing a polar side chain, selected from the group His, Arg, Lys, citrulline, ornithine, 2,3-diaminopropionic acid, 2,4-diaminobutyric acid, and (S)-2-amino-4-guanindinobutyric acid, or, Trp;
AA4 is an amino acid containing an aromatic side chain, selected from the group Trp, Phe, Tyr, 1-naphthylalanine, 2-naphthylalanine and phenylglycine;
(x, y, z, b) = 1 or 2, where the indices x, y, z, b are integers that can adopt values 1 or 2, representing the number of units of, respectively, AA1, AA2, AA3 and AA4;
R1 is an N-terminal chain modifier selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
R2 is selected from the group consisting of -NR3R4, -OR3 and -SR3, where R3 and R4 are independently selected from the group consisting of H, substituted or unsubstituted acyclic aliphatic, substituted or unsubstituted cyclic aliphatic, substituted or unsubstituted heterocyclic, substituted or unsubstituted heteroarylalkyl, substituted or unsubstituted aryl, substituted or unsubstituted arylalkyl, substituted or unsubstituted acyl, substituted or unsubstituted carbonyl;
their stereoisomers, mixtures and/or their cosmetically or pharmaceutically acceptable salts.

2. Compound, according to claim 1, **characterized in that**:
AA1 is Pro;
AA2 is Thr, Trp, Tyr or 1-naphthylalanine, 2-naphthylalanine and phenylglycine;
AA3 is Lys or Trp;
AA4 is Trp, Lys or 1-naphthylalanine.
wherein R1 is either Pal-, which identifies an N- palmitoylated peptide, or Ac-, which identifies an N-acetylated peptide.

3. Compound according to claim 1, **characterized by** being Pro- Trp - Lys- Trp (SEQ NO ID 1), Pal -Pro- Thr -Lys- Trp (SEQ NO ID 2), Ac-Pro- Trp - Lys- Trp (SEQ NO ID 3), Pro-1-naphthyl Ala-Lys- 1-naphthyl Ala (SEQ NO ID 4), Pal -Pro- Trp -Lys- Trp (SEQ NO ID 5), Pro- Thr -Lys- Trp (SEQ NO ID 6), Ac-Pro- Thr -Lys- Trp (SEQ NO ID 7)
wherein the prefix Pal - identifies an N- palmitoylated peptide and the prefix Ac- identifies an N-acetylated peptide.

4. Compound, according to claim 1, **characterized in that** it is for muscle relaxation, including the skeletal muscles of the face, for anti-aging cosmetic action, for reducing and/or smoothing expression lines and wrinkles and/or for antiperspirant action in an individual.

5. Cosmetic, dermocosmetic, cosmeceutical or pharmaceutical composition, **characterized by** comprising at least one compound as defined in any one of claims 1 to 4.

6. Composition according to claim 5, **characterized in that** the concentration of the compound is between 0.001% (w/w) and 10.00% (w/w).

7. Composition according to claim 5 or 6, **characterized in that** it is for anti-aging cosmetic action, for reducing and/or smoothing expression lines and wrinkles and/or for antiperspirant action in an individual.

8. Use of a composition as defined in any one of claims 5 to 7, **characterized in that** it is for the production of cosmetic, dermocosmetic, cosmeceutical or pharmaceutical formulations for the relaxation of muscles, including the skeletal muscles of the face, for anti-aging cosmetic action, for reducing and/or smoothing expression lines and wrinkles and/or for antiperspirant action in an individual.

9. Cosmetic method for relaxing the skeletal muscles of the face, for anti-aging cosmetic action, for reducing and/or smoothing expression lines and wrinkles and/or for antiperspirant action, **characterized by** comprising the application of an effective cosmetic amount of a composition as defined in any one of claims 5 to 7 to an individual.

10. Method for dermocosmetic, cosmeceutical or pharmaceutical for relaxing the skeletal muscles of the face, for anti-aging cosmetic action, for reducing and/or smoothing expression lines and wrinkles and/or for antiperspirant action **characterized by** comprising the application of an effective cosmetic amount of a composition as defined in any one of claims 5 to 7 to an individual.
